(19) **Europäisches Patentamt**
**European Patent Office**
**Office européen des brevets**

(11) **EP 4 334 692 B1**

(12) **EUROPEAN PATENT SPECIFICATION**

(45) Date of publication and mention
of the grant of the patent:
**09.04.2025  Bulletin 2025/15**

(21) Application number: **22720523.4**

(22) Date of filing: **28.04.2022**

(51) International Patent Classification (IPC):
$G01K\ 17/20\ ^{(2006.01)}$        $G01W\ 1/17\ ^{(2006.01)}$

(52) Cooperative Patent Classification (CPC):
**G01K 17/20; G01W 1/17**

(86) International application number:
**PCT/NL2022/050226**

(87) International publication number:
**WO 2022/235153 (10.11.2022 Gazette 2022/45)**

(54) **THERMAL COMFORT MEASURING SYSTEM**

SYSTEM ZUR MESSUNG DES THERMISCHEN KOMFORTS

SYSTÈME DE MESURE DE CONFORT THERMIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **04.05.2021  NL 2028142**

(43) Date of publication of application:
**13.03.2024  Bulletin 2024/11**

(73) Proprietor: **Hukseflux Holding B.V.
2628 XJ Delft (NL)**

(72) Inventor: **VAN DEN BOS, Cornelis Jan
2628 XJ Delft (NL)**

(74) Representative: **Nederlandsch Octrooibureau
P.O. Box 29720
2502 LS The Hague (NL)**

(56) References cited:
**EP-A1- 1 455 171        EP-A2- 3 568 639
US-A- 4 906 105          US-A- 5 216 625
US-A1- 2020 189 352**

- **MEGGERS FORREST ET AL: "The
Thermoheliodome - "Air conditioning" without
conditioning the air, using radiant cooling and
indirect evaporation", ENERGY, vol. 157, pages
11 - 19, XP085263526, ISSN: 0378-7788, DOI:
10.1016/J.ENBUILD.2017.06.033**

- **MEGGERS FORREST ET AL: "Improving Mean
Radiant Temperatures Sensing Using
Multidirectional Non-Contacting Temperature
Sensors to Avoid Convective Errors With Globe
Thermometers", 2022 IEEE INTERNATIONAL
WORKSHOP ON METROLOGY FOR LIVING
ENVIRONMENT (METROLIVEN), IEEE, 25 May
2022 (2022-05-25), pages 18 - 22, XP034152159,
[retrieved on 20220718], DOI: 10.1109/
METROLIVENV54405.2022.9826986**

- **ANONYMOUS: "USER MANUAL - TCOMSYS01
Hot Cube - Thermal comfort measuring system",
HUKSEFLUX CORPORATE WEBSITE, 5
November 2018 (2018-11-05), Delftechpark 31,
2628 XJ Delft (The Netherlands), XP055936194,
Retrieved from the Internet <URL:https://www.
hukseflux.com/uploads/product-documents/
TCOMSYS01_manual_v2003.pdf> [retrieved on
20220628]**

- **ANONYMOUS: "USER MANUAL - FHF01 - Foil
heat flux sensor, flexible, 50 x 50 mm, with
temperature sensor", HUKSEFLUX CORPORATE
WEBSITE, 25 April 2017 (2017-04-25),
Delftechpark 31, 2628 XJ Delft (The Netherlands),
XP055936204, Retrieved from the Internet
<URL:https://www.hukseflux.com/uploads/
product-documents/FHF01_manual_v2006.pdf>
[retrieved on 20220628]**

## Description

### Technical field

The invention relates to a heat flux sensor comprising a body with six or more sensor pairs, each pair consisting of one radiation absorbing sensor for measuring a combined radiative and convective heat flux and one radiation reflecting sensors for measuring a convective heat flux.

The invention also relates to a system comprising such a heat flux sensor and to a method of determining a thermal comfort value, in particular a predicted mean vote (PMV).

### Background

Thermal comfort is defined by ISO 7730 clause 7 as that condition of mind which expresses satisfaction with the thermal environment and is assessed using subjective evaluation. The 7730 standard presents methods to predict thermal sensation and degree of discomfort of people exposed to moderate thermal environments, like in homes, offices and cars. The methods are centered around the determination of the Predicted Mean Vote (PMV) and the associated Predicted Percentage of Dissatisfied (PPD). Knowing the PMV, the PPD can directly be calculated. One of the ways to determine the PMV is by direct measurement, using an integrating sensor. The concept of an integrating sensor refers to a measuring instrument performing the measurement of several variables needed to determine thermal comfort.

An important parameter in thermal comfort is the mean radiant temperature. It is defined as the uniform temperature of an imaginary enclosure in which radiant heat transfer from the human body is equal to the radiant heat transfer in the actual non-uniform enclosure.

ISO 7726 mentions in Clause 4.1.2 that the black globe thermometer is a device frequently used to derive an approximate value of the mean radiant temperature from the globe temperature, $T_g$, and the temperature and the velocity of the air surrounding the globe. It also points out that the measurement with the black globe is a major source of uncertainty of the PMV measurement. Large corrections need to be applied. These corrections depend on the air velocity, i.e. the estimate of the velocity of natural or forced convection, which needs to measured separately. In many cases the air velocity is not measured and thus not known, which leads to a larger measurement uncertainty than attainable with a known air velocity or direct heat flux measurements. A benefit of the globe thermometer is that it measures in all directions; it measures an integrated "mean" radiant temperature.

ISO 7726 clause 4.1.2 indicates that the spherical shape of the globe thermometer, integrating approximately over a solid angle of $4\pi$ sr, is a reasonable approximation of a seated person, but that an ellipsoidal shape would in fact be preferrable for the upright and seated positions.

In general ISO 7726 indicates that the mean radiant temperature may also be calculated from measured values if the plane radiant temperature in different directions is weighed according to the projected area factors for a person. In such case, multiple measurements are made with a sensor facing relevant directions, and the measured data are post-processed to arrive at the desired result. Calculating PMV for a certain situation, may require a procedure specific to the case, for example repositioning the sensor to point in different directions, and calculation based on scientific judgement, typically calculating an average or weighed average of the measurements, for example according to annex B of ISO 7726.

ISO 7726 further mentions in clause 4.1.2 that determining mean radiant temperature ideally requires measurements in 6 opposite directions and weighing these according to the projected area factors (optical view factors) for a person. Annex B on Mean Radiant Temperature comments on projected area factors in 6 directions (up-down, left-right, front-back, pairwise perpendicular axes) for thermal comfort estimation of persons in standing and seated positions. These projected area factors indicate the relative contribution of these directions for radiative exchange. Table B.1 of ISO 7726 shows that for a seated person all 6 directions are approximately equally relevant, while for a standing person the up-down directions make a smaller contribution than the others.

Although this is not specifically highlighted in ISO 7726, a measurement method of thermal comfort should also include a measurement of convective heat transfer representative of convective heat transfer to the human body. A common and reasonable assumption is that the projected area factors which are applicable to radiative heat flux are also valid for convective heat flux.

A black globe thermometer or a ellipsoid body, ISO 7726 assumes, does not only exchange radiative energy but also convective energy in a way representative of the human body when seated. It seems reasonable to conclude that a thermal comfort sensor, capable of determining heat fluxes in one measurement, without repositioning, should have a view angle of substantially 2.5 $\pi$ to $4\pi$ sr approximately equally sensitive in all 6 directions.

In US 2020/0189352, a vehicle with a one-directional thermal comfort sensor system is shown which includes an air velocity sensor, an air temperature sensor, a radiant heat flux sensor and a humidity sensor on one side of a headrest of a vehicle seat to sense the passenger compartment. Using this data, a climate controller calculates a current PMV-value of thermal comfort using the data from the sensor system and the data from a biometric sensor wearable by the passenger. A climate control system is further included for heating or cooling the exterior surface of the sensor system.

Operative temperature is defined as the uniform temperature in which an occupant would exchange the same amount of heat as in the existing non-uniform environment. Operative temperature is a practical way of expressing overall thermal exposure.

The invention aims to provide a reliable heat flux sensor and measurement system that can produce a more accurate determination of the heat fluxes at the sensor, that can provide an accurate operative temperature and that in particular can provide an accurate determination of the thermal comfort. It is also an object of the invention to produce a measurement immediately representative of the directional response of a human body in the situation that is investigated, taking projected area factors into account for persons in different positions without re-positioning the sensor. It is also an object of the invention to provide a versatile measurement system with which the need for separate air velocity measurements is obviated. It is a further aim to provide a sensor and a system in which the contribution of the different individual parameters that result in thermal comfort or discomfort, can be accurately determined.

Heat flux sensors are known in the art. For instance, TCOMSYS01 is a measuring system to help understand and quantify "causes and effect" leading to human thermal comfort. The TCOMSYS01 body is temperature stabilised at 33°C, so that it offers a relatively direct measurement of the human experience. In essence, TCOMSYS01 is a miniature thermal mannequin, measuring according to the innovative Hot Cube method. The heater power required to keep the TCOMSYS01 at a constant temperature is the main measurand. Incorporating 5 heat flux sensors with a black absorber, it also offers a detailed picture of the heat gain and loss from different directions, and a good indication of convective and radiative asymmetry. Other measurements are sensor body temperature, air temperature and relative humidity. Further details can be found in the document "USER MANUAL - TCOMSYS01 Hot Cube - Thermal comfort measuring system" on the Hukseflux corporate website.

**Summary of the invention**

Hereto a heat flux sensor according to the invention comprises the features of claim 1

The sensor according to the invention is able to separately measure radiant and convective heat transfer from six or more sides of the sensor body, which may be made of metal. By its geometry it is capable of measuring heat transfer representative of that to a human body.

Typical shapes of the body are rectangular or cylindrical. In case of a rectangular body typically six sides are equipped with heat flux sensor pairs. In case of a cylindrical body, sensor pairs are typically deposited on 90° of the circular cross section and possibly on the top and bottom as well. A general requirement is that their combined field of view is between $2.5\pi$ to $4\pi$ sr.

The absorptive (typically black coated) heat flux sensor measures radiative as well as convective heat flux, whereas the reflective (typically gold-coated) heat flux sensor measures convective heat flux only. By subtracting the 2 measured fluxes, the radiative flux can be determined, the convective heat transfer coefficient $C_{tr}$ can be determined and the ambient air velocity can be calculated. No separate air velocity measurement or estimate thereof is required. By separately determining all these factors, conclusions can be drawn about their contribution to the thermal comfort and about whether a specific factor is a cause or an effect.

The heat flux sensor also contains a body temperature sensor such as a thermocouple of thermistor and a heating member, such that the sensor can measure heat fluxes at multiple different sensor body temperatures. The body temperatures can be controlled to an estimated clothing temperature, which makes it possible to measure the exact heat loss at this clothing temperature. When local circumstances change, the temperature of the body can be adapted rapidly to the new conditions, contrary to the use of a known black globe sensor.

The heating member must have the capability of heating the sensor body to a substantially uniform temperature. For example, at 32 °C steady state body temperature at an air temperature between 15 and 20 °C and lower than 1 m/s air speed (typical office conditions), the temperature difference between sensors should remain below 2 °C, so that 2 sensor pairs per axis facing in substantially opposite directions are substantially equally sensitive to radiative and convective heat flux. At heat flux level in the order of 100 W/m$^2$, and heat transfer coefficients combined radiative and convective heat flux in the order of 10 (W/m$^2$)/C, a 2 °C error creates a 20 % error, which is rated acceptable.

By measuring at more than 2 body temperature levels, the quality control and assurance of the thermal comfort measurements may be improved. Quality assurance of the heat flux measurements is also obtainable by comparing the total power in W required to keep the sensor at a stable temperature to the measured heat fluxes in W/m$^2$ and the sensor body surface area in m$^2$.

By first determining radiative and convective transfer coefficients, and then controlling the temperature of the body to a stable temperature, for example the clothing temperature, the operative temperature can be determined.

An embodiment of a heat flux sensor according to the invention comprises at least six differently oriented surfaces, each carrying a absorptive sensor and a reflective sensor.

By equipping multiple sides of the body with heat flux sensor pairs, the heat fluxes from different directions can be determined. By equipping the body with an even number of sensor pairs mounted with their fields of view in opposite directions, each having a field of view between $2\pi$ to $4\pi$ sr, imbalances or radiant and convective asymmetry can simultaneously be detected.

The measured heat fluxes, combined other measured parameters and parameters entered by the user, make it possible to calculate the PMV and PPD score for the different sides, in particular 6 sides, of the body and an overall integrated PMV and PPD score, which is based on the combined heat fluxes.

A sensor for which the combined solid angle is between $2.5\pi$ sr and $4\pi$ sr may also act as an integrating sensor, approximating the solid angle and directional response of a human body. Results for seated and standing persons may also be calculated using mathematical weighing per axis as proposed by ISO 7726 Annex B.

The sensor body and sensors mounted on it may also be curved, for example cylindrical, having the same directional properties - that is sensitivity to radiative and convective fluxes - as an approximate cylindrical human body at the position of the sensor.

Using two sensor pairs mounted in opposite directions, in case of radiant or convective asymmetry, the sensor allows detection of such asymmetry and will be able to issue a warning, in a case in which the use of a known black globe thermometer could give to a "false positive" indication of a comfortable situation.

A system according to the invention comprises the heat flux sensor, a control unit and an ambient temperature sensor connected to the control unit for measuring an ambient temperature $T_{air}$, the control unit being adapted for:

- operating the heating member for substantially uniform heating of the body,
- determining or controlling of the temperature of the body, $T_{sen}$,
- determining from the measurements of the reflective sensor of each of the six or more sensor pairs a convective heat flux $\Phi_{convection}$,
- determining a radiative heat flux $\Phi_{radiation}$ by subtracting the measurements of the absorptive sensors and of the reflective sensor of each of the six or more sensor pairs,
- **implementing** corrections to improve measurements, for example correcting $\Phi_{convection}$, and $\Phi_{radiation}$ case emission and reflection of sensor coatings deviate significantly from 1,
- implementing mathematical corrections such as the projected area factors valid for the person under study
- determining a convective heat transfer coefficient $C_{tr}$ based on $\Phi_{convection}$, $T_{air}$ and $T_{sen}$ or based on the power supplied to the heating member at several sensor body temperatures $T_{sen}$,
- determining an ambient air velocity $v_{air}$, based on the heat transfer coefficient $C_{tr}$ and
- determining the radiative temperature $T_{rad}$ from $T_{sen}$ and $\Phi_{radiation}$

The system may have an active temperature control of the sensor body wherein the control unit controls the power supplied to the heating member so that the sensor body is kept at a predetermined temperature $T_{sen}$.

In the system according to the invention, the heat transfer coefficient $C_{tr}$ can be determined by

$$C_{tr} = \Phi_{convection} / ( T_{air} - T_{sen} ),$$

and the air velocity $v_{air}$ can be determined from $C_{tr}$, using an empirical formula valid for the sensor geometry and the direction of the ambient air flow, for example by :

$$v_{air} = ((C_{tr} - B) / A)^2$$

wherein A and B are constants.

The constants A and B are typically empirically determined.

It is also possible to use average values of $\Phi_{convection}$ in several directions to determine the air velocity. A correction may be made for non-perfect radiation absorption (i.e. absorption deviating from 1) and non-perfect reflection (i.e. reflection deviating from 1).

An embodiment of a system according to the invention further comprises a humidity sensor connected to the control unit for determining a humidity $p_a$ of the ambient air, the control unit being adapted for determining a comfort value for a person at the position of the sensor, based on:

- the measured humidity $p_a$ of the ambient air,
- the body temperature, $T_{sen}$,
- measured air temperature, $T_{air}$,
- for each of the six or more sensor pairs, the radiative heat flux $\Phi_{radiation}$, and the radiative and the convective heat flux $\Phi_{convection}$.

- a single measurement with a sensor having a representative directional response and field of view for the person at the position of the sensor or
- a single measurement with a sensor not having a representative directional response and field of view for the person under study, and using mathematical corrections such as the projected area factors valid for the person under study
- a series of measurements with a sensor not having a representative directional response and field of view for the person under study, repositioning the sensor to point in different directions, and calculating a result from these measurements based on scientific judgement ISO 7730 expresses thermal comfort using the predicted mean vote, PMV, which is linked to the PPD index, predicted percentage dissatisfied. The PMV has a 7 -point scale; hot, warm, slightly warm, neutral, slightly cool, cool and cold. From the PMV the predicted percentage dissatisfied, PPD, can be calculated. The PPD has a minimum of 5 % when PMV = 0 (neutral) and reaches 75 % when PMV= $\pm$ 2 (warm or cool). A person is considered to be dissatisfied when he or she considers the environment hot, warm, cool or cold.

In more detail: PMV is a function of 6 input parameters; metabolic rate M in $W/m^2$, clothing insulation $I_{cl}$, air temperature $T_{air}$, mean radiant temperature $T_{rad}$, relative air velocity $v_{air}$ and the water vapor partial pressure $p_a$ in Pa .

One standard metabolic unit (met) corresponds to 58.2 $W/m^2$. The metabolic rate for a sedentary activity is considered to be 1.2 met = 70 $W/m^2$. The clothing insulation $I_{cl}$ is expressed in $K·m^2/W$. One standard clothing unit (clo) is 0.155 $K·m^2/W$, which corresponds to a typical set of garment worn in a working environment.

According ISO 7730 the PMV can be calculated, within a certain rated operating range defining "moderate conditions", most importantly $v_{air}$ < 1 m/s, M < 4 met, $I_{cl}$:< 2 clo, $T_{air}$ 10 to 30 °C by:

$$PMV = [0.303\ e^{(-0.036\ M)} + 0.028] \times [\ [(M-W) - 3.05 \times 10^{-3}\ [5733 - 6.99\ (M-W) - p_a] - 0.42\ [(M-W) - 58.15] - 1.7 \times 10^{-5}\ M\ (5867 - p_a) - 0.0015\ M\ (34 - T_{air}) - 3.96\ 10^{-8}\ f_d\ ((T_{cl} + 273)^4 - (T_{rad} + 273)^4) - f_{cl}\ C_{tr}\ (T_d - T_{air})\ ]$$

**With** $T_{cl}$ **the** clothing temperature:

$$T_{cl} = 35.7 - 0.028\ (M-W) - I_{cl}\ [3.96\ 10^{-8}\ f_{cl}\ ((T_{cl} + 273)^4 - (T_{rad} + 273)^4) + f_{cl}\ C_{tr}\ (T_{cl} - T_{air})]$$

The second term between brackets in the equation for PMV represents the heat balance between the human being and its surrounding. If the balance is negative, this will result in a cool or cold sensation. The first five factors of the heat balance describe the heat loss by evaporation, by sweating and by respiration. Since the largest part of the human body is covered and insulated by clothing, the heat losses through radiation and convection are calculated at the clothing surface, using its estimated surface temperature, $T_{cl}$. The sixth and seventh component of the heat balance gives the linearized heat loss through radiation, the eight component the heat loss through convection. The radiation model assumes that 71 % of the body area acts as a net emitter of radiative energy with an emissivity of 0.97. The remaining 29 % of the area, for example between the legs, absorbs as much as it emits, resulting in a zero balance. These factors are included in the multiplier of 3.96 $10^{-8}$ of the sixth component (0.97 x 0.71 x 5.77 x $10^{-8}$ = 3.96 x $10^{-8}$) .

In this equation, $T_{cl}$ is the clothing temperature, W the effective mechanical power, $C_{tr}$ the convective heat transfer coefficient, $f_{cl}$ clothing surface area factor: ratio of the surface area of the clothed body to the nude body. The effective mechanical power produced is usually set at 0 W, assuming office work or driving a car. This needs to be adapted if heavy work is done.

In a typical analysis, the user determines a typical metabolic rate, clothing surface area factor and the clothing insulation. As a next step $T_{cl}$ must then be estimated.

In known systems, the black globe thermometer is a device frequently used to derive an approximate value of the mean radiant temperature from the globe temperature, $T_g$, and the temperature and the velocity of the air surrounding the globe. The measurement with the black globe is a major source of uncertainty of the PMV measurement that leads to large corrections being applied. These corrections depend on the air velocity, i.e. the estimate of the velocity of natural or forced convection, which is in the known systems usually not measured and thus not known. This inaccuracy is prevented with the system according to the invention.

Contrary to known black globe thermometer systems, and other systems described in ISO 7726 and 7730, the system according to the invention measures the heat loss through convection and radiation directly.

Knowing the ratio between these two heat fluxes makes it possible to accurately estimate, using the above formula, the $T_{cl}$. This reduces the PMV measurement uncertainty relative to the crude estimate open to users of the black globe thermometer. The air velocity measurement or assumptions about air velocity are no longer needed; fluxes are directly measured.

In addition, the effect of projected area factors in 4 or 6 different directions for humans in different positions such as standing and seated, can be taken into account per surface in the terms $(T_{rad} + 273)^4$ and $C_{tr}\ (T_{cl} - T_{air})$.

A sensor for which the combined solid angle is between $2.5\pi$ sr and $4\pi$ sr may also act as an integrating sensor, approximating the solid angle and directional response of a human body. The known black globe thermometer, which has the shape and directional response of a sphere, integrates approximately over $4\pi$ sr.

In an embodiment of the sensor, the sensor body and sensors mounted are example cylindrical, having the same directional properties - that is sensitivity to radiative and convective fluxes - as an approximately cylindrical human body under study.

In its preferred system embodiment, the sensor body temperature is controlled to the clothing temperature, and the PMV and PPD are determined with a sensor body temperature at or around $T_{cl}$. It is possible to measure the PMV also if the body temperature of the measuring system is not at the calculated clothing (surface) temperature. For the measurement, we assume that the heat flux is a linear function of the body temperature and zero at ambient air temperature. At a small difference between actual and target temperature (a few degrees), the error in PMV is small.

Contrary to black globe thermometer systems, the present measuring system allows users to draw conclusions about cause and effect of discomfort. The black globe only issues a number. This discomfort is typically caused by unwanted cooling or heating of a particular part of the body; system users can separate the contributions of radiation, convection, and air velocity.

In an embodiment of a system according to the invention, the control unit is adapted to compare the total heating power supplied to the body for keeping the body at a predetermined temperature, with the heat flux measured by the reflective sensor and the heat flux measured by the absorptive sensor of each of the six or more sensor pairs. This can provide a quality benchmark for the flux measurements.

In another embodiment of a system according to the invention, it measures $C_{tr}$ and during the measurement the sensor body is controlled to a stable temperature, for example the appropriate clothing temperature $T_{cl}$ to accurately measure the convective and radiative flux at that temperature. This results in a high accuracy calculation of the operative temperature.

Alternatively, the operative temperature may be determined by the control unit heating the body at a number of different temperatures, and determining from the radiative heat fluxes $\Phi_{radiation}$ and the convective heat fluxes $\Phi_{convection}$ at these temperatures, and from those the radiative and convective heat transfer coefficients and an operative temperature.

In case the control unit determines a comfort value for different surfaces of the sensor body, mounted with their fields of view in opposite directions, it can provide an indication if the difference in comfort values measured at these surfaces is above a predetermined threshold value. In case of radiant or convective asymmetry, the sensor allows detection of such asymmetry and will be able to issue a warning.

The system according to the invention can also provide an indication, such as a visual sign or a sound or flagging of stored records, when the air velocity $v_{air}$ exceeds a predetermined threshold value. For accurate PMV measurements, it is preferred that the air velocity is below 1 m/s as air speeds above this value generally result in discomfort. The indication provided by the system prevents in this case that invalid PMV values are attributed.

**Brief description of the drawings**

Figure 1 shows a perspective view of a sensor for measuring a heat flux not in accordance with the present invention;

Figure 2 shows a cross-sectional side view of a measurement side of the sensor for measuring a heat flux not in accordance with the present invention;

Figure 3 shows a perspective view of a measurement side of the sensor for measuring a heat flux not in accordance with the present invention;

Figure 4 shows a perspective view of a cylindrical sensor for measuring a heat flux not in accordance with the present invention;

Figure 5 shows a perspective view of a sensor for measuring a heat flux in accordance with the present invention;

Figure 6 schematically shows a system not according to the invention for measuring thermal comfort;

Figure 7 shows a flowchart of the steps carried out by the control unit for determining thermal comfort. in accordance with the present invention.

## Detailed description of embodiments

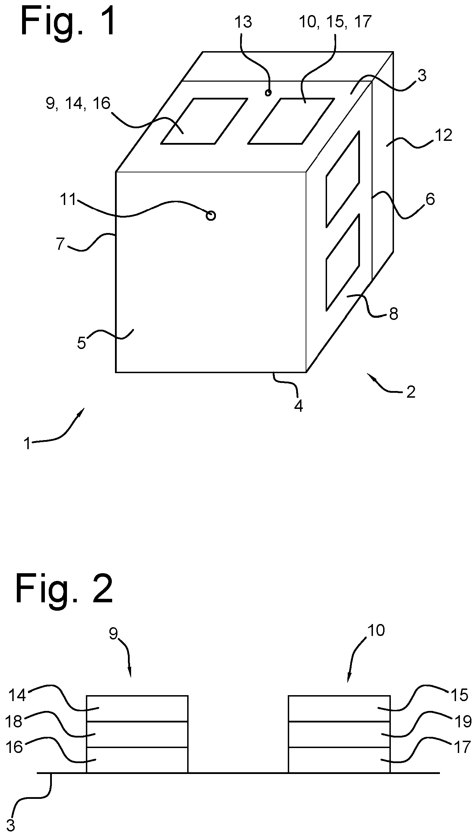

Figure 1 depicts a sensor that is not covered by the scope of the claims. Figure 1 shows a perspective view of a sensor 1 for measuring a heat flux. The sensor comprises a body 2 with a plurality of sides 3, 4, 5, 6, 7, 8, an absorptive heat flux sensor 9 operable to absorb thermal radiation and a reflective heat flux sensor 10 operable to reflect thermal radiation. The sensor may optionally be equipped with an ambient air temperature sensor 13. The body 2 comprises at least one temperature sensor 11 for measuring the temperature of the body and at least one heating member 12. In this case the two sides 3 and 8 (not visible) each contain a sensor pair, the opposite sides 4 and 7 also contain a sensor pair.

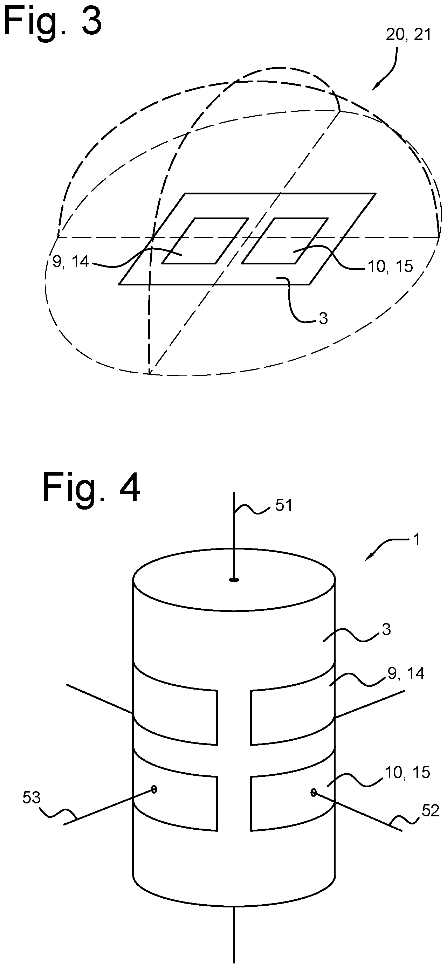

The absorptive heat flux sensor 9 operable to absorb thermal radiation is further referred to as absorptive sensor. This absorptive sensor 9 measures both convective and thermal radiation. The reflective heat flux sensor 10 operable to reflect thermal radiation is further referred to as reflective sensor. The reflective sensor 10 measures predominantly convective heat flux. The body 2 is well conducting heat, typically made of metal with a thermal conductivity of larger than 10 W/(m·K) and a heat capacity larger than 18 J / K typically made of metals such as aluminium, brass or steel. Each side of the body 3, 4, 5, 6, 7, 8 is a predominantly flat surface with a different spatial orientation. Four sides 3, 4, 8, 7 of the body 2 are equipped with the absorptive sensor 9, the reflective sensor 10 and are further referred to as measurement sides 3, 4, 7, 8. The absorptive sensor 9 and the reflective sensor 10 on the measurement sides 3, 4, 7, 8 have a substantially similar mounting surface area and field of view as further described in figure 3, for the respective absorption and reflection of thermal radiation to be comparable and the sensitivity to convection to be comparable. The absorptive and reflective sensors may be placed anywhere on the measurement sides 3, 4, 7, 8, as long they have a substantially similar exposure of their surface area to thermal radiation and convection. The absorptive sensor 9 is typically black coated to absorb thermal radiation, the coating may be made of a black high temperature paint qualifying as high absorptivity layer 14 the heat flux sensor may be made using a thermopile sensing element encapsulated in a plastic 16, or another method measuring a temperature difference across a solid layer and presents a thermal absorptivity between 0.8 and 1. The absorptive sensor 9 measures both a convective and a radiative heat flux, $\Phi_{convection} + \Phi_{radiation}$, through its surface on the measurement side 3. The reflective sensor 10 is typically gold coated to reflect thermal radiation, may be made of a gold, silver, aluminium or nickel layer qualifying as high reflectivity layer 14 on a heat flux sensing element 17 and presents a thermal reflectivity between 0.5 and 1. Heat flux sensing elements 16, 17 convert the thermal energy of absorbed radiation for each sensor into an output signal, in case of a thermopile and electrical voltage. The reflective sensor 10 measures predominantly a convective flux $\Phi_{convection}$ through its surface on the measurement sides 3, 4, 7, 8.

By subtracting the heat flux measured by the reflective sensor 10, $\Phi_{convection}$, from the heat flux measured by the absorptive sensor 9, $\Phi_{convection} + \Phi_{radiation}$, through their surfaces of substantially equal sensitivity to convective heat flux on the measurement sides 3, 4, 7, 8, the radiative heat flux $\Phi_{radiation}$ may be estimated. The sensor 1 is therefore able to separately measure radiant and convective heat transfer on the measurement side 3. Mathematical corrections may be applied for reflectivity and absorptivity not equal to 1. The effect of projected area factors for humans in different positions such as standing and seated, can be taken into account per sensor side.

The heating member 12 is positioned in heat conducting contact with the body 2 and may be used to heat the body 2 to a predetermined temperature or at a predetermined power. Due to the body's high thermal conductivity, typically made of aluminium, brass or steel, combined with its mass, expressed as heat capacity, it internally has a low thermal resistance and the body is uniformly heated to the predetermined temperature, $T_{sen}$, via heat conduction from the heating member.

The heating member must have the capability of heating the sensor body to a substantially uniform temperature, so that two sensor pairs per axis facing in substantially opposite directions are equally sensitive to radiative and convective heat flux.

The temperature sensor 11 thermally coupled with the body 2 is used to measure the temperature of the body. By controlling the body 2 to a predetermined temperature via the heating member 12 and the temperature sensor 11, radiative and convective heat transfer to an object of that particular temperature $T_{sen}$, relevant to a user, can be simulated and measured. By measuring heat transfer using the absorptive sensor 9 and the reflective 10 sensor at multiple temperature levels of the body 2, from $\Phi$ convection, $_1$, $\Phi$ convection, $_2$ at two or more predetermined temperatures $T_{sen, 1}$, $T_{sen, 2}$, the air temperature $T_{air}$ and heat transfer coefficient $C_{tr}$ can both be calculated, eliminating the need for a separate air temperature measurement, and improving the level of quality assurance of the heat transfer measurements:

$$C_{tr} = (\Phi_{convection,2} - \Phi_{convection, 1}) / (T_{sen, 1} - T_{sen, 2})$$

with positive heat flux from surrounding environment to the sensor,
and/or

- calculate an ambient air temperature $T_{air}$ at or near the sensor by :

$$T_{air} = T_{sen,\ 1} + \Phi_{convection,\ 1} \left( T_{sen,\ 1} - T_{sen,\ 2} \right) / \left( \Phi_{convection,\ 2} - \Phi_{convection,\ 1} \right)$$

The optional ambient air temperature sensor 13 can be also used to separately measure an air temperature. An ambient air velocity $v_{air}$ can further be calculated from the heat transfer coefficient using an empirical formula valid for the sensor geometry and the direction of the ambient air flow for example according to:

$$v_{air} = ((C_{tr} - B)/A)^2$$

where A, B are constants empirically determined or taken from theory.

Figure 2 shows a cross-sectional side view of the measurement plane 3, 4, 8 or 7 of the sensor 1 for measuring a heat flux of Figure 1. The absorptive sensor 9 comprises, besides the high absorptivity layer 14 and the thermopile sensing element 16, a calibration heating element 18. Furthermore the reflective sensor 10 comprises, besides the high reflectivity layer 15 and the heat flux sensing element 17, a calibration heating element 19.

Each calibration heating element 18, 19 is placed between the high absorptivity layer 14 and the thermopile sensing element 16 or between the high reflectivity layer 15 and the heat flux sensing element 17, respectively. Calibration heating elements 18, 19 may be identical and/or built integrally within the absorptive and reflective sensors 9, 10. The sensitivity, surface area and electrical resistance of the calibration heating elements 18, 19 are predetermined. They allow calculation of a measured calibration heat flux for a measured output voltage over or current through the calibration heating element.

By incorporating calibration heating elements 18, 19 on the heat flux sensors 9, 10, the sensors can be calibrated using a simple and unique procedure for both sensors, where the ratio of the measured calibration heat flux, $\Phi_c$, to an output signal U of the thermopile or heat flux sensing element 16, 17 can be used to calculate a sensitivity E of the absorptive sensor and the reflective sensor 9, 10 according to:

$$E = U/\Phi_c$$

In other embodiments, alternative calibration functions may also be used. Heat fluxes may also be corrected for losses to the environment, edge effects etc. During calibration the heat flux sensor surfaces may be thermally insulated. The emissivity and absorption of the sensor surfaces may be separately estimated by inspection or by experiment.

Figure 3 shows a perspective view of the measurement surface plane 3, 4, 8 or 7 of the sensor 1 for measuring a heat flux of Fig. 1. The predominantly flat measurement surface 3, 4, 8 or 7 is equipped with an absorptive sensor 9 and a reflective sensor 10 disposed at any location on the measurement surface and comprising the high absorptivity layer 14 and the high reflectivity layer 15, respectively, exposed to environmental heat fluxes. Each of the absorptive and reflective sensors 9, 10 have a substantially similar field of view 20, 21. Each field of view 20, 21 expands substantially 180 degrees in a longitudinal and in a transverse direction in an entrance plane at the measurement side 3, in other words a solid angle of $2\pi$ sr.

Figure 4 depicts a sensor that is not covered by the scope of the claims. Figure 4 shows a perspective view of a sensor body 1 where the body is a cylinder and the sensors, 9, 10 comprising the high absorptivity layer 14 and the high reflectivity layer 15 are deposited on 90 ° of the circular cross section. In contrast, in an embodiment of the invention comprising six sensors, they may as well be deposited on the top and bottom of the cylinder. A general requirement is that their combined field of view is between $2.5\pi$ to $4\pi$ sr. The drawing also shows examples of pairwise perpendicular axes 51, 52, and 53 representing 6 directions of a person: up-down, left-right and front-back.

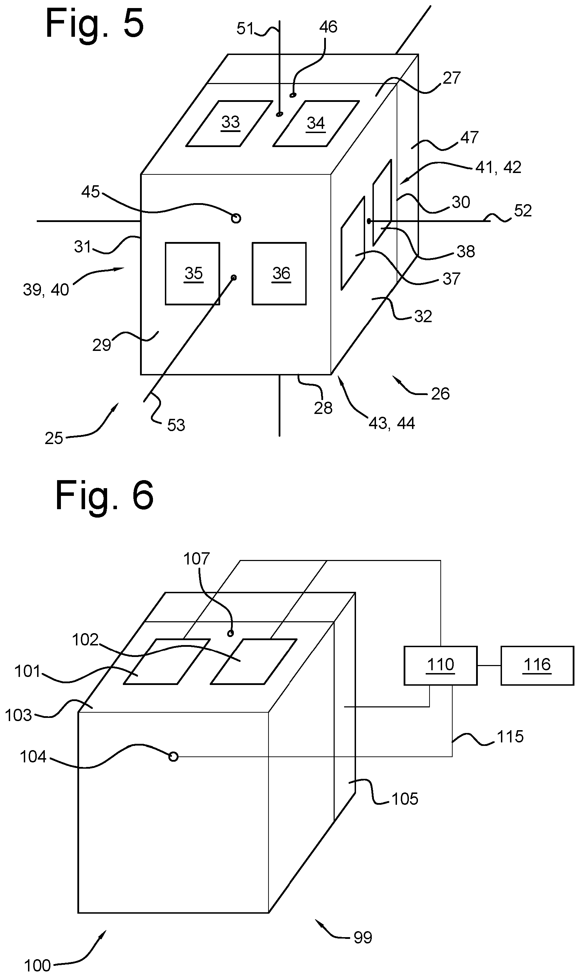

Figure 5 shows a perspective view of a sensor 25 for measuring a heat flux in accordance with an embodiment of the present invention. The sensor comprises a body 26 with a plurality of sides 27, 28, 29, 30, 31, 32, at least six absorptive heat flux sensors 33, 35, 37, 39, 41, 43 operable to absorb thermal radiation and at least six reflective heat flux sensors 34, 36, 38, 40, 42, 44 operable to reflect thermal radiation. The sensor 25 may optionally be equipped with an ambient air temperature sensor 46. The body 26 comprises at least one body temperature sensor 45 and at least a heating member 47.

The at least six absorptive heat flux sensors 33, 35, 37, 39, 41, 43 operable to absorb thermal radiation are further referred to as absorptive sensors, and the at least six reflective heat flux sensors 34, 36, 38, 40, 42, 44 operable to reflect thermal radiation are further referred to as reflective sensors. They function identically and may be placed identically as the absorptive and reflective sensors described in the embodiment of Figure 1, where each side 27, 28, 29, 30, 31, 32 is also a flat surface with a different spatial orientation. Further the absorptive sensor and the reflective sensor have also a substantially similar mounting plane and field of view as previously described in Figures 3 and 4. In the present embodiment at least six sides 27, 28, 29, 30, 31, 32 are each equipped with an absorptive sensor, a reflective sensor and are further referred to as measurement sides., for the respective sensitivities to convective heat flux and absorption and reflection of thermal radiation to be comparable. The sensors may also be calibrated with respective calibration heating element such as shown previously in Figure 2.

The heating member 47 is positioned in heat conducting contact with the body 26 and may be used to heat the body 26 to a predetermined temperature, $T_{sen}$. Due to the body's thermal conductivity, typically made of aluminium, brass or steel, and internally low thermal resistance the body is uniformly heated to the predetermined temperature via heat conduction from the heating member. The temperature sensor 45 thermally coupled with the body 26 may be used to measure the temperature of the body. By controlling the body 26 to a predetermined temperature via the heating member 47 and the temperature sensor 46, radiative and convective heat transfer to an object of that particular temperature with surfaces of different orientations 27, 28, 29, 30, 31, 32, relevant to a user, can be simulated and measured for each orientation.

By measuring heat transfer using the absorptive sensor 33, 35, 37, 39, 41, 43 and the reflective sensors 34, 36, 38, 40, 42, 44 at multiple temperature levels of the body 26, from the convective heat values $\Phi_{convection, 1}$, $\Phi_{convection, 2}$ at two or more predetermined temperatures $T_{sen, 1}$, $T_{sen, 2}$, the air temperature $T_{air}$ and heat transfer coefficient $C_{tr}$ can both be calculated in the vicinity of each the at least two measurement sides, eliminating the need for a separate air temperature measurement, and improving the level of quality assurance of the heat transfer measurements. Furthermore the ambient air velocity $v_{air}$ near each of the at least six measurement sides, can also be determined as in the sensor of Fig. 1.

The drawing also shows examples of pairwise perpendicular axes 51, 52, and 53 representing 6 directions of a person: up-down, left-right and front-back.

Figure 6 depicts a sensor that is not covered by the scope of the claims. Figure 6 shows a system 99 for measuring thermal comfort, comprising a body 100, with the absorptive heat flux sensor 101 and the reflective heat flux sensor 102 situated on the same face 103 and having substantially similar fields of view between $1.5\pi$ and $3\pi$ sr. A temperature sensor 104, such as a thermocouple, for measuring the temperature of the body 100 is placed in heat conducting contact with the body 100. A control unit 110 is connected via control lines 115 to a heater 105 and temperature sensor 104 for heating the body 100 to a predetermined temperature, and to a temperature sensor and humidity sensor 107 for measuring the temperature and humidity of the ambient air. A terminal 116 is connected to the control unit 110. The terminal 116 comprises a user interface for the input of user commands and data to the control unit 110.

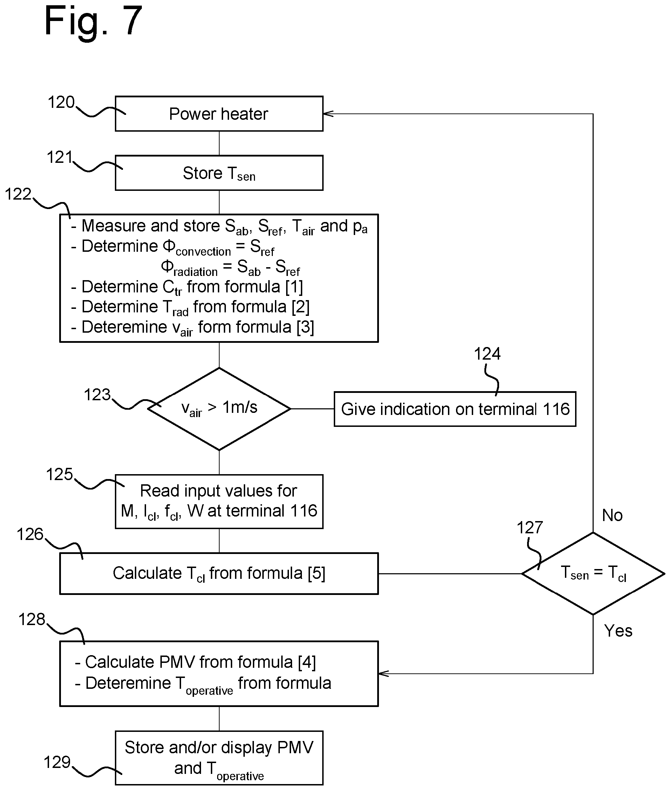

Figure 7 shows a method of determining a thermal comfort value, in particular a predicted mean vote PMV. The control unit 110 comprises a processor and memory unit that calculate and store the PMV value and the operative temperature at the sensor body 100 by the following steps.

The heater 105 is powered to set the temperature of the body 100 and sensors 101, 102 to a predetermined temperature, for example $T_{cl}$ in step 120. The sensor temperature $T_{sen}$ is stored in step 121. In step 122:

- the heat flux sensor signal $S_{ab}$ of absorptive sensor 101 and the heat flux sensor $S_{ref}$ of reflective sensor 102 are measured and stored in the memory of the control unit 110,
- the air temperature signal $T_{air}$ and the water vapour partial pressure signal $p_a$ of the humidity sensor are measured and stored in the memory of the control unit 110,
- The convective heat flux $\Phi_{convection}$ is determined from the sensor value $S_{ref}$ of reflective sensor 102: $\Phi$ convection = $S_{ref}$,
- the radiative heat flux $\Phi_{radiation}$ is determined from the difference of the sensor value $S_{ab}$ of the absorptive sensor 101 and the sensor value $S_{ref}$ of the reflective sensor 102: $\Phi_{radiation}$ = $S_{ab}$-$S_{ref}$,
- the convective heat transfer coefficient of the air, $C_{tr}$ is calculated by Formula 1, and
- the air velocity $v_{air}$ is determined by Formula 5.

Corrections can be implemented in step 122 to improve measurements, for example correcting $\Phi_{convection}$, and $\Phi_{radiation}$ in case the emission and reflection of sensor coatings deviate significantly from 1, or taking the effect of projected area factors for humans in different positions such as standing and seated, into account.

In step 123 it is determined if $v_{air}$ > 1m/s. If this is so, a digital, optical or acoustic warning signal is generated by the control unit 110 in step 124.

In step 125, the values for the metabolic rate, M, clothing isolation $I_{cl}$, clothing factor $f_{cl}$, the effective mechanical power W and a radiative heat transfer coefficient are entered by the user in the terminal 116 and read into the memory of the control unit 110.

In step 126:

- the clothing temperature $T_{cl}$ is calculated by the control unit 110 by Formula 5,
- the sensor body is controlled to $T_{cl}$, as long as $T_{sen}$ is not within a pre-set limit from $T_{sen}$, steps 120 to 125 may be repeated via step 127.

In step 128

- the PMV value is calculated by Formula 4 and
- the operative temperature, $T_{operative}$, is calculated by Formula 7.

- radiant an convective asymmetries are calculated by subtracting heat flux values from sensors mounted with their fields of view in opposite directions.
- a PMV may be calculated from a series of measurements possibly in different direction calculating a result from these measurements based on scientific judgement

The values of PMV and $T_{operative}$ can be stored in the memory of the control unit 110 and/or displayed on a display of terminal 116 in step 129.

**Thermal Comfort**

ISO 7730 clause 7 defines: Thermal comfort is that condition of mind which expresses satisfaction with the thermal environment and is assessed using subjective evaluation.

The 7730 standard presents methods to predict thermal sensation and degree of discomfort of people exposed to moderate thermal environments, like in homes, offices and cars. The methods are centered around the determination of the Predicted Mean Vote (PMV) and the associated Predicted Percentage of Dissatisfied (PPD). One of the ways to determine the PMV, described in clause 4.1, is by direct measurement, using an integrating sensor.

ISO 7726 mentions in clause 4.1.2 that determining heat fluxes ideally requires measurements in 6 opposite directions and weighing these according to the projected area factors (optical view factors) for a person. Annex B on Mean Radiant Temperature comments on projected area factors in 6 directions (up-down, left-right, front-back, pairwise perpendicular axes) for thermal comfort estimation of persons in standing and seated positions. These projected area factors indicate the relative contribution of these directions for radiative exchange. Table B.1 of ISO 7726 shows that for a seated person all 6 directions are approximately equally relevant, while for a standing person the up-down directions make a smaller contribution than the others.

ISO 7726 mentions in Clause 4.1.2 that the black globe thermometer is a device frequently used to derive an approximate value of the mean radiant temperature from the globe temperature, $T_g$, and the temperature and the velocity of the air surrounding the globe. It also points out that the measurement with the black globe is a major source of uncertainty of the PMV measurement. Large corrections are applied. These corrections depend on the air velocity, i.e. the estimate of the velocity of natural or forced convection, which is usually not measured and thus not known, which leads to a larger measurement uncertainty than attainable with a known air velocity or direct heat flux measurements.

Instead of black globe sensor, the present invention provides a new thermal comfort measuring system 99 based on measurement of radiative and convective heat fluxes with at least six sensor pairs such as 101, 102 to the temperature controlled sensor body 100.

The thermal comfort measuring system is able to separately measure radiant and convective heat transfer from one or more sides of a (metal) heat transfer sensor body 100. To do so, at least six sides of the sensor body 100 is equipped with the heat flux sensor pair such as 101, 102: an absorptive (typically black coated) heat flux sensor 101, which measures radiative as well as convective heat flux, and a reflective (typically gold-coated) heat flux sensor 102, which measures convective heat flux only. By subtracting the 2 fluxes measured by the sensors 101,102 in the control unit 110, the radiative flux may be estimated.

Preferably, the temperature of the sensor body 100 is controlled by means of the heater 106, temperature sensor 104 and the control unit 110, so that the system 99 can measure heat fluxes at the sensor pair 101, 102 at multiple different body temperatures.

The heating member must have the capability of heating the sensor body to a substantially uniform temperature. For example, at 32 °C steady state body temperature at an air temperature between 15 and 20 °C and lower than 1 m/s air speed (typical office conditions), the temperature difference between sensors should remain below 2 °C, so that two sensor pairs per axis facing in substantially opposite directions are equally sensitive to radiative and convective heat flux.

The heat fluxes in W/m$^2$, the body temperature in °C, possibly combined with the power in W supplied to the heater 105 and needed to maintain a fixed body temperature, and the ambient air temperature measured by the sensor 107 in °C, are used in the control unit 110 to calculate all local parameters determining thermal transport, the fluxes caused by radiation and convection and the heat transfer coefficient.

In combination with a temperature and humidity measurement of the ambient air in % measured by the sensor 107, and user-entered parameters specific to the situation that is investigated the system 99 according to the invention can be used to predict thermal sensation and degree of discomfort (PMV and PPD) as described by ISO 7730, ASHRAE Standard 55 and CEN CR 1752. The complete system is then considered an "integrating sensor to directly measure the thermal comfort" as described in ISO 7730, paragraph 4.1.

When measuring at a known sensor body temperature, if the ambient air temperature is also known, or from measurements at multiple body temperatures, the local heat transfer coefficient can be estimated. From the heat transfer coefficient, the local air velocity can be calculated.

The system 99 can also be used to directly measure operative temperature. This can be done by controlling the sensor body 100 to a stable temperature, for example the clothing temperature and using the measured heat flux values at that temperature, or by calculation after performing multiple measurements at different sensor body temperatures, measured by the sensor 104 and set in the control unit through powering of the heater 105. If needed the radiative and convective transfer coefficients can be corrected to account for the properties of the "occupant", resulting in an adapted estimate.

**System physics**

The black coated heat flux sensor 101 measures $\Phi_{radiation} + \Phi_{convection}$. The gold coated heat flux sensor 102 measures $\Phi_{convection}$ only. By subtraction, $\Phi_{radiation}$ can be calculated.

The sensor body can be operated at a certain constant power in W or be controlled to a certain body temperature in °C.

The convective heat transfer coefficient of the air, with positive heat fluxes from surrounding environment (air) to the sensor, is calculated by:

$$C_{tr} \qquad = \Phi_{convection} / (T_{air} - T_{sen}) \qquad\qquad [1]$$

The radiative temperature is:

$$T_{rad} = (T^4_{sen} + \Phi_{radiative} / (\varepsilon\,\sigma))^{1/4} \qquad\qquad [2]$$

with $\varepsilon$ the absorption of the sensor coating and $\sigma$ the Stefan Boltzmann constant.

The ambient air velocity can be estimated, from $C_{tr}$ using [1] and empirical formula's such as:

$$v_{air} = ((C_{tr} - B) / A)^2 \qquad\qquad [3]$$

In such empirical formula's the constants A and B may be empirically determined. Typical orders of magnitude for a single perpendicularly exposed heat flux sensor surface: A = 36.7 and B = - 1.25. For surfaces exposed to air flow at 45°: A = 30. 0 and B = -0.78.

It is also possible to use average values of $\Phi_{convection}$ under several directions relative to the airflow to determine the air velocity.

The electrical power consumption of the heater 106 at one or more body temperature levels of the body 100, may also be used to estimate the heat transfer coefficient.

**PMV, PPD measurements**

ISO 7730 expresses thermal comfort using the predicted mean vote, PMV, which is linked to the PPD index, predicted percentage dissatisfied. The PMV has a 7 -point scale; hot, warm, slightly warm, neutral, slightly cool, cool and cold. From the PMV the predicted percentage dissatisfied, PPD, can be calculated. The PPD has a minimum of 5 % when PMV = 0 (neutral) and reaches 75 % when PMV= $\pm$ 2 (warm or cool). A person is considered to be dissatisfied when he or she considers the environment hot, warm, cool or cold.

In more detail: PMV is a function of 6 input parameters; metabolic rate M in W/m$^2$, clothing insulation $I_{cl}$, air temperature $T_{air}$, mean radiant temperature $T_{rad}$, relative air velocity $v_{air}$ and the water vapour partial pressure $p_a$ in Pa.

One standard metabolic unit (met) corresponds to 58.2 W/m$^2$. The metabolic rate for a sedimentary activity is considered to be 1.2 met = 70 W/m$^2$. The clothing insulation $I_{cl}$ is expressed in K·m$^2$/W. One standard clothing unit (clo) is 0.155 K·m$^2$/W, which corresponds to a typical set of garment worn in a working environment.

According ISO 7730, the PMV can be calculated, within a certain rated operating range defining "moderate conditions", most importantly $v_{air}$ < 1 m/s, M < 4 met, $I_{cl}$:< 2 clo, $T_{air}$ 10 to 30 °C.

$$PMV = [0.303\ e^{(-0.036\ M)} + 0.028] \times$$

$$[\qquad [(M-W)- 3.05 \times 10^{-3}\ [5733 - 6.99\ (M-W) - p_a]\ -0.42\ [(M-W)\ -58.15]$$

$$- 1.7 \times 10^{-5}\ M\ (5867 - p_a) - 0.0015\ M\ (34 - T_{air})$$

$$- 3.96\ 10^{-8}\ f_{cl}\ ((T_{cl} + 273)^4 - (T_{rad} + 273)^4)$$

$$- f_{cl}\ C_{tr}\ (T_{cl} - T_{air}) \qquad\qquad ] \qquad [4]$$

The second term represents the heat balance between the human being and its surrounding. If the balance is negative, this will result in a cool or cold sensation. The first and second lines of the heat balance describe the heat loss by evaporation, by sweating and by respiration.

Since the largest part of the human body is covered and insulated by clothing, the heat losses through radiation and convection are calculated at the clothing surface, using its estimated surface temperature, $T_{cl}$.

The third component of the heat balance gives the linearized heat loss through radiation, the fourth component the heat loss through convection. The radiation model assumes that 71 % of the body area acts as a net emitter of radiative energy with an emissivity of 0.97. The remaining 29 % of the area, for example between the legs, absorbs as much as it emits, resulting in a zero balance. These factors are included in the multiplier of $3.96 \cdot 10^{-8}$ of the third component ($0.97 \times 0.71 \times 5.77 \times 10^{-8} = 3.96 \times 10^{-8}$).

In this equation, $T_{cl}$ is the clothing temperature, W the effective mechanical power, $C_{tr}$ the convective heat transfer coefficient, $f_{cl}$ clothing surface area factor: ratio of the surface area of the clothed body to the nude body. The effective mechanical power produced is usually set at 0 W, assuming office work or driving a car. This needs to be adapted (through user input via the terminal 116) if heavy work is done.

In a typical analysis, the user determines a typical metabolic rate, clothing surface area factor and the clothing insulation. These data are entered into the control unit 110 via the terminal 116. As a next step, in the control unit 110, $T_{cl}$ must then be estimated by iteration, using the 3.96 factor mentioned above again to correct radiative view factors:

$$T_{cl} = 35.7 - 0.028 \, (M-W) - I_{cl} \, [3.96 \cdot 10^{-8} \, f_{cl} \, ((T_{cl}+ 273)^4 - (T_{rad}+273)^4) + f_{cl} \, C_{tr} \, (T_{cl}-T_{air})] \quad [5]$$

Operative temperature $T_{operative}$ is defined in ISO 7730 as: the uniform temperature of an imaginary black enclosure, and the air within it, in which an occupant would exchange the same amount of heat by radiation plus convection as in the actual nonuniform environment.

In ISO 7726, appendix G2 gives the exact equation for a sensor for which the directional response is representative of the human body, or using a series of measurements to determine a representative heat flux. Measuring $\Phi_{radiation, \, cl}$ and $\Phi_{convection, \, cl}$, using a sensor with its body stabilised at one or more temperatures for example $T_{cl}$, allows accurate calculation of $T_{operative}$:

$$T_{operative} = T_{cl} + ( \Phi_{radiation, \, cl} + \Phi_{convection, \, cl} ) / (C_{tr} + C_{rad}) \quad [7]$$

## Claims

1. Heat flux sensor (1, 25) comprising:

   a body (2, 26) with six or more sensor pairs, each pair consisting of one radiation absorbing, absorptive, sensor (9, 33, 35) for measuring a combined radiative and convective heat flux and one radiation reflecting, reflective, sensor (10, 34, 36) for substantially measuring a convective heat flux,
   the body exhibiting three axes of heat flux measurement, the axes being substantially perpendicular, with two sensor pairs per axis facing substantially in opposite directions,
   a heating member (12, 47) that is in heat conducing contact with the body, capable of heating the body uniformly within 2 °C for body temperatures between 25 and 40 °C at ambient air temperatures between 20 and 25 °C at air speeds < 1 m/s and
   a temperature sensor (11, 45) thermally coupled with the body for measuring the body temperature $T_{sen}$.

2. Heat flux sensor according to claim 1, carrying six or more sensor pairs with a combined field of view (20, 21) between $2.5\pi$ to $4\pi$ sr,

3. Heat flux sensor according to claim 1 and 2, carrying six or more sensor pairs mounted with their fields of view in opposite directions, each having a field of view between $2\pi$ to $4\pi$ sr,

4. System comprising the heat flux sensor (1, 25, 100) of claim 1 or 2 or 3, a control unit (116) and an ambient temperature sensor (13, 46, 107) connected to the control unit for measuring an ambient temperature $T_{air}$, the control unit being adapted for:

   - operating the heating member (12, 47, 105) for heating of the body,
   - determining or controlling of the temperature of the body, $T_{sen}$

- determining from the measurements of the reflective sensor of each of the six or more sensor pairs a convective heat flux $\Phi_{convection}$,
- determining a radiative heat flux $\Phi_{radiation}$ by subtracting the measurements of the absorptive sensor and of the reflective sensor of each of the six or more sensor pairs,
- determining a convective heat transfer coefficient $C_{tr}$ based on $\Phi_{convection}$, $T_{air}$ and $T_{sen}$ or based on the power supplied to the heating member at several sensor body temperatures $T_{sen}$,
- determining an ambient air velocity $v_{air}$, based on the heat transfer coefficient $C_{tr}$, and
- determining the radiative temperature $T_{rad}$ from $T_{sen}$ and $\Phi_{radiation}$.

5. System according to claim 4, wherein the control unit controls the power supplied to the heating member so that the sensor body is kept at a predetermined temperature $T_{sen}$ .

6. System according to claim 4 or 5 wherein the heat transfer coefficient $C_{tr}$ is determined by

$$C_{tr} = \Phi_{convection} / ( T_{air} - T_{sen} ),$$

and the air velocity $v_{air}$ is determined using an empirical formula from $C_{tr}$, the empirical formula being valid for a geometry of the sensor and a direction of the ambient air flow, for example by :

$$v_{air} = ((C_{tr} - B) / A)^2,$$

wherein A and B are constants; wherein the control unit provides an indication when the air velocity $v_{air}$ exceeds a predetermined threshold value.

7. System according to claim 4, or 5 or 6, further comprising a humidity sensor (107) connected to the control unit for determining a humidity $p_a$ of the ambient air, the control unit being adapted for determining a comfort value for a person at the position of the sensor, based on:

- the measured humidity $p_a$ of the ambient air,
- the measured air temperature, $T_{air}$,
- the body temperature, $T_{sen}$,
- for each of the six or more sensor pairs, the radiative heat flux $\Phi_{radiation}$, and the convective heat flux $\Phi_{convection}$.
- a single measurement with a sensor according to claims 1 or 2 or 3 having a representative directional response and field of view for the person at the position of the sensor or
- a single measurement with a sensor not having a representative directional response and field of view for the person at the position of the sensor, and using mathematical corrections such as projected area factors valid for the person at the position of the sensor or
- a series of measurements with sensors according to claims 1 or 2 or 3, not having a representative directional response and field of view for the person at the position of the sensor, repositioning the sensor to point in different directions, and calculating a result from these measurements

8. System according to claim 7, wherein the comfort value comprises a predicted mean vote PMV which is a function of parameters including a metabolic rate M, that can be expressed in $W/m^2$, the effective mechanical power W, that can be expressed in $W/m^2$, clothing insulation $I_{cl}$, that can be expressed in $K \cdot m^2/W$, the air temperature $T_{air}$, a mean radiant temperature $T_{rad}$, that can be expressed in $^\circ C$, the ambient air velocity $v_{air}$, that can be expressed in m/s and the measured humidity $p_a$ that can be expressed by water vapor partial pressure in Pa, which parameters are related:

$$PMV = [0.303\ e^{\,(-0.036\ M)} + 0.028] \times$$
$$[\ [(M-W) - 3.05 \times 10^{-3}\ [5733 - 6.99\ (M-W) - p_a] - 0.42\ [(M-W) - 58.15]$$
$$- 1.7 \times 10^{-5}\ M\ (5867 - p_a) - 0.0015\ M\ (34 - T_{air}) - 3.96\ 10^{-8}\ f_{cl}\ ((T_{cl} + 273)^4 - (T_{rad} + 273)^4) -$$
$$f_{cl}\ C_{tr}\ (T_{cl} - T_{air})\ ],$$

Wherein values of M, W, $I_{cl}$, $f_{cl}$ are entered by the user, and $f_{cl}$ is a clothing surface area factor, and $T_{cl}$ the clothing surface temperature:

$$T_{cl} = 35.7 - 0.028 \ (M\text{-}W) - I_{cl} \ [3.96 \ 10^{-8} \ f_{cl} \ ((T_{cl}+ 273)^4 - (T_{rad}+273)^4) + \ f_{cl} \ C_{tr} \ (T_{cl}\text{-}T_{air})]$$

9. System according to claim 4, wherein the control unit controls the power supplied to the heating member so that the sensor body is kept at $T_{cl}$. and PMV and PPD values are measures at or around $T_{cl}$

10. System according to any of claims 4 to 9, the control unit being adapted to compare the total heating power supplied to the body for keeping the body at the predetermined temperature, with the heat flux measured by the reflective sensor and the heat flux measured by the absorptive sensor of each of the six or more sensor pairs.

11. System according to any of claims 4-10, the control unit heating the body to one or more stable temperatures for example $T_{cl}$, thereby determining the operative temperature $T_{operative}$:

$$T_{operative} = T_{cl} + ( \ \Phi_{radiation, \ cl} + \Phi_{convection, \ cl} ) \ / \ (C_{tr} + C_{rad} )$$

with the subscript "cl" indicating at clothing surface temperature and $C_{rad}$ the radiative heat transfer coefficient.

12. System according to any of claims 4-11, the control unit heating the body at a number of different temperatures, and determining from the radiative heat fluxes $\Phi_{radiation}$ and the convective heat fluxes $\Phi_{convection}$ at these temperatures an air temperature, radiative and convective heat transfer coefficients, and an operative temperature.

13. System according to claims 8 or 9 in combination with claim 2, having 2 or another even number of sensor pairs, wherein the control unit determines a comfort value, radiative heat flux of convective heat flux for each surface, and provides an indication if a difference in comfort value, radiative heat flux or convective heat flux is above a predetermined threshold value.

14. Method of determining a comfort value, in particular a predicted mean vote PMV, the method comprising the steps of:

   - providing a heat flux sensor (100) having a body (99) with six or more sensor pairs, each sensor pair consisting of one absorptive sensor (101) for measuring a combined radiative and convective heat flux and one reflective sensor (102) for substantially measuring a convective heat flux, a heating member (105) that is in heat conducing contact with the body, a temperature sensor (104) thermally coupled with the body for measuring the body temperature $T_{sen}$, and a humidity sensor (107) connected to the control unit for determining a humidity $p_a$ of the ambient air, and an ambient temperature sensor (107) connected to the control unit for measuring an ambient temperature $T_{air}$,
   - operating the heating member for heating of the body (120),
   - determining the temperature of the body, $T_{sen}$ ,
   - determining for each of the six or more sensor pairs from the measurements of the reflective sensor a convective heat flux $\Phi_{convection}$,
   - determining for each of the six or more sensor pairs a radiative heat flux $\Phi_{radiation}$ by subtracting the measurements of the absorptive sensor and of the reflective sensor,
   - determining a convective heat transfer coefficient $C_{tr}$ based on $\Phi_{convection}$, $T_{air}$ and $T_{sen}$ or based on the power supplied to the heating member at several sensor body temperatures $T_{sen}$, and
   - determining an ambient air velocity $v_{air}$, based on the heat transfer coefficient $C_{tr}$,
   - entering values of M metabolic rate, W work, $I_{cl}$, clothing insulation, $f_{cl}$ clothing surface area factor and

   determining the comfort value based on:

   - the measured humidity $p_a$ of the ambient air,
   - the measured air temperature, $T_{air}$,
   - the body temperature, $T_{sen}$,
   - for each of the six or more sensor pairs the radiative heat flux $\Phi_{radiation}$, and the convective heat flux $\Phi_{convection}$.
   - a single measurement with a sensor according to claims 1 and 2 having a representative directional response and field of view for the person at the position of the sensor or
   - a series of measurements with sensors according to claims 1 and 2 not having a representative directional response and field of view for the person at the position of the sensor, repositioning the sensor to point in different directions, and calculating a result from these measurements

15. Method according to claim 14, wherein the comfort value comprises a predicted mean vote PMV which is a function of parameters including a metabolic rate M, that can be expressed in $W/m^2$, the effective mechanical power W, that can be expressed in $W/m^2$, clothing insulation $I_{cl}$, that can be expressed in $K \cdot m^2/W$, the air temperature $T_{air}$, a mean radiant temperature $T_{rad}$, that can be expressed in C, the ambient air velocity $v_{air}$, that can be expressed in m/s and the measured humidity $p_a$ that can be expressed by water vapor partial pressure in Pa, which parameters are related by:

$$PMV = [0.303\, e^{(-0.036\, M)} + 0.028] \times [\,[(M-W) - 3.05 \times 10^{-3} [5733 - 6.99 (M-W) - p_a] - 0.42 [(M-W) - 58.15] - 1.7 \times 10^{-5} M (5867 - pa) - 0.0015\, M (34 - Tair) - 3.96\; 10^{-8} f_d ((T_{cl} + 273)^4 - (T_{rad} + 273)^4) - f_{cl}\, Ctr\, (T_{cl} - T_{air})\,],$$

Wherein values of M, W, $I_{cl}$, $f_{cl}$ are entered by the user, and $f_{cl}$ is a clothing surface area factor, and $T_{cl}$ the clothing surface temperature

$$T_{cl} = 35.7 - 0.028\, (M-W) - I_{cl}\, [3.96\; 10^{-8}\; f_{cl} ((T_{cl} + 273)^4 - (T_{rad} + 273)^4) + f_{cl}\, C_{tr}\, (T_{cl} - T_{air})]$$

**Patentansprüche**

1. Wärmestromsensor (1, 25), umfassend:

   einen Körper (2, 26) mit sechs oder mehr Sensorpaaren, wobei jedes Paar aus einem strahlungsabsorbierenden, Absorptionssensor (9, 33, 35) zur Messung eines kombinierten Strahlungs- und Konvektionswärmestroms und einem strahlungsreflektierenden, Reflexionssensor (10, 34, 36) zur wesentlichen Messung eines Konvektionswärmestroms besteht,
   der Körper drei Achsen zur Messung des Wärmestroms aufweist, wobei die Achsen im Wesentlichen senkrecht sind und zwei Sensorpaare pro Achse im Wesentlichen in entgegengesetzte Richtungen weisen,
   ein Heizelement (12, 47), das in wärmeleitendem Kontakt mit dem Körper steht und dazu eingerichtet ist, den Körper gleichmäßig innerhalb von 2 °C für Körpertemperaturen zwischen 25 und 40 °C bei Umgebungslufttemperaturen zwischen 20 und 25 °C bei Luftgeschwindigkeiten < 1 m/s zu erwärmen,
   und einen Temperatursensor (11, 45), der thermisch mit dem Körper gekoppelt ist, um die Körpertemperatur $T_{sen}$ zu messen.

2. Wärmestromsensor nach Anspruch 1, der sechs oder mehr Sensorpaare mit einem kombinierten Sichtfeld (20, 21) zwischen $2{,}5\pi$ und $4\pi$ sr trägt.

3. Wärmestromsensor nach Anspruch 1 und 2, der sechs oder mehr Sensorpaare trägt, die mit ihren Sichtfeldern in entgegengesetzte Richtungen angebracht sind und jeweils ein Sichtfeld zwischen $2\pi$ und $4\pi$ sr aufweisen.

4. System umfassend den Wärmestromsensor (1, 25, 100) nach Anspruch 1 oder 2 oder 3, eine Steuereinheit (116) und einen mit der Steuereinheit verbundenen Umgebungstemperatursensor (13, 46, 107) zur Messung einer Umgebungstemperatur $T_{air}$, wobei die Steuereinheit dazu eingerichtet ist:

   - Betreiben des Heizelements (12, 47, 105) zur Erwärmung des Körpers,
   - Bestimmen oder Kontrollieren der Körpertemperatur, $T_{sen}$
   - Bestimmen eines Konvektionswärmestroms $\Phi_{convection}$ aus den Messungen des Reflexionssensor jedes der sechs oder mehr Sensorpaare,
   - Bestimmen eines Strahlungswärmestroms $\Phi_{radiation}$ durch Subtraktion der Messwerte des Absorptionssensors und des Reflexionssensors jedes der sechs oder mehr Sensorpaare,
   - Bestimmen eines konvektiven Wärmeübergangskoeffizienten $C_{tr}$ auf der Grundlage von $\Phi_{convection}$, $T_{air}$ und $T_{sen}$ oder auf der Grundlage der dem Heizelement bei mehreren Sensorkörpertemperaturen $T_{sen}$ zugeführten Leistung,
   - Bestimmen einer Umgebungsluftgeschwindigkeit $v_{air}$ auf der Grundlage des Wärmeübergangskoeffizienten $C_{tr}$ und
   - Bestimmen der Strahlungstemperatur $T_{rad}$ aus $T_{sen}$ und $\Phi_{radiation}$.

5. System nach Anspruch 4, wobei die Steuereinheit die dem Heizelement zugeführte Leistung so steuert, dass der Sensorkörper auf einer vorgegebenen Temperatur $T_{sen}$ gehalten wird.

6. System nach Anspruch 4 oder 5, wobei der Wärmeübergangskoeffizient $C_{tr}$ bestimmt wird durch

$$C_{tr} = \Phi_{convection} / ( T_{air} - T_{sen} ) , \text{ und}$$

die Luftgeschwindigkeit $v_{air}$ mit Hilfe einer empirischen Formel aus $C_{tr}$ bestimmt wird, wobei die empirische Formel für eine Geometrie des Sensors und eine Richtung der Umgebungsluftströmung gültig ist, z. B. durch:

$$v_{Air} = ((C_{tr} - B) / A)^2,$$

wobei A und B Konstanten sind; wobei die Steuereinheit eine Meldung ausgibt, wenn die Luftgeschwindigkeit $v_{air}$ einen vorgegebenen Schwellenwert überschreitet.

7. System nach Anspruch 4 oder 5 oder 6, das ferner einen Feuchtigkeitssensor (107) umfasst, der mit der Steuereinheit verbunden ist, um eine Feuchtigkeit $p_a$ der Umgebungsluft zu bestimmen, wobei die Steuereinheit dazu eingerichtet ist, einen Komfortwert für eine Person an der Position des Sensors zu bestimmen, basierend auf:

- der gemessenen Feuchtigkeit $p_a$ der Umgebungsluft,
- der gemessenen Lufttemperatur $T_{air}$,
- der Körpertemperatur $T_{sen}$,
- dem Strahlungswärmestrom $\Phi_{radiation}$ und dem Konvektionswärmestrom $\Phi_{convection}$ für jedes der sechs oder mehr Sensorpaare.
- einer Einzelmessung mit einem Sensor nach einem der Ansprüche 1 oder 2 oder 3, der eine repräsentative Richtungsempfindlichkeit und Sichtfeld für die Person an der Position des Sensors aufweist, oder
- einer Einzelmessung mit einem Sensor, der keine repräsentative Richtungsempfindlichkeit und Sichtfeld für die Person an der Position des Sensors aufweist, und unter Verwendung mathematischer Korrekturen wie projizierter Flächenfaktoren, die für die Person an der Position des Sensors gelten, oder
- einer Reihe von Messungen mit Sensoren nach einem der Ansprüche 1 oder 2 oder 3, die keine repräsentative Richtungsempfindlichkeit und Sichtfeld für die Person an der Position des Sensors aufweisen, wobei der Sensor so umpositioniert wird, dass er in verschiedene Richtungen zeigt, und ein Ergebnis aus diesen Messungen berechnet wird.

8. System nach Anspruch 7, wobei der Komfortwert eine erwartete durchschnittliche Empfindung PMV umfasst, die eine Funktion von Parametern ist, einschließlich einer Stoffwechselrate M, die in $W/m^2$ ausgedrückt werden kann, der effektiven mechanischen Leistung W, die in $W/m^2$ ausgedrückt werden kann, der Bekleidungsisolierung $I_{cl}$, die in K. $m^2/W$ ausgedrückt werden kann, die Lufttemperatur $T_{air}$, eine mittlere Strahlungstemperatur $T_{rad}$, die in °C ausgedrückt werden kann, die Umgebungsluftgeschwindigkeit $v_{air}$, die in m/s ausgedrückt werden kann, und die gemessene Luftfeuchtigkeit $p_a$, die durch den Wasserdampfpartialdruck in Pa ausgedrückt werden kann, welche Parameter miteinander in Beziehung stehen:

$$PMV = [0{,}303 \; e^{(-0.036 \, M)} + 0{,}028] \; x$$

$$[ \; [(M-W) - 3{,}05 \times 10^{-3} [5733 - 6{,}99 \, (M-W) - p_a] - 0{,}42 \, [(M-W) - 58{,}15]$$

$$- 1{,}7 \times 10^{-5} \, M \, (5867 - p_a) - 0{,}0015 \, M \, (34 - T_{air}) - 3{,}96 \cdot 10^{-8} \, f_{cl} \, ((T_{cl} + 273)^4 - (T_{rad} + 273)^4)$$

$$- f_{cl} \, C_{tr} \, (T_{cl} - T_{air}) \; ],$$

wobei die Werte M, W, $I_{cl}$ und $f_{cl}$ vom Benutzer eingegeben werden, und wobei $f_{cl}$ ein Faktor für die Oberfläche der Kleidung und $T_{cl}$ die Oberflächentemperatur der Kleidung ist:

$$T_{cl} = 35{,}7 - 0{,}028 \, (M-W) - I_{cl} \, [3{,}96 \cdot 10^{-8} \, f_{cl} \, ((T_{cl} + 273)^4 - (T_{rad} + 273)^4) + f_{cl} \, C_{tr} \, (T_{cl} - T_{air})]$$

9. System nach Anspruch 4, wobei die Steuereinheit die dem Heizelement zugeführte Leistung so steuert, dass der Sensorkörper auf $T_{cl}$ gehalten wird und die PMV- und PPD-Werte bei oder um $T_{cl}$ gemessen werden.

10. System nach einem der Ansprüche 4 bis 9, wobei die Steuereinheit dazu eingerichtet ist, die gesamte dem Körper zugeführte Heizleistung, um den Körper auf der vorgegebenen Temperatur zu halten, mit dem vom Reflexionssensor gemessenen Wärmestrom und dem vom Absorptionssensor gemessenen Wärmestrom jedes der sechs oder mehr Sensorpaare zu vergleichen.

11. System nach einem der Ansprüche 4-10, wobei die Steuereinheit den Körper auf eine oder mehrere stabile Temperaturen, z. B. $T_{cl}$, erwärmt und dadurch die operative Temperatur $T_{operative}$ bestimmt:

$$T_{operative} = T_{cl} + ( \Phi_{radiation,\ cl} + \Phi_{convection,\ cl} ) / ( C_{tr} + G_{rad} )$$

wobei der Index "cl" die Oberflächentemperatur der Kleidung und $C_{rad}$ den Strahlungswärmeübergangskoeffizienten angibt.

12. System nach einem der Ansprüche 4-11, wobei die Steuereinheit den Körper auf eine Anzahl verschiedener Temperaturen erwärmt und aus den Strahlungswärmeströmen $\Phi_{radiation}$ und den Konvektionswärmeströmen $\Phi_{convection}$ bei diesen Temperaturen eine Lufttemperatur, Strahlungs- und Konvektionswärmeübergangskoeffizienten und eine Betriebstemperatur bestimmt.

13. System nach Anspruch 8 oder 9 in Kombination mit Anspruch 2, mit 2 oder einer anderen geraden Anzahl von Sensorpaaren, wobei die Steuereinheit einen Komfortwert, einen Strahlungswärmestrom eines Konvektionswärmestroms für jede Oberfläche, bestimmt und eine Anzeige bereitstellt, wenn ein Unterschied im Komfortwert, im Strahlungswärmestrom oder im Konvektionswärmestrom über einem vorgegebenen Schwellenwert liegt.

14. Verfahren zur Bestimmung eines Komfortwerts, insbesondere einer erwarteten durchschnittlichen Empfindung PMV, wobei das Verfahren die folgenden Schritte umfasst:

- Bereitstellen eines Wärmestromsensors (100) mit einem Körper (99) mit sechs oder mehr Sensorpaaren, wobei jedes Sensorpaar aus einem Absorptionssensor (101) zum Messen eines kombinierten Strahlungs- und Konvektionswärmestroms und einem Reflexionssensor (102) zum im Wesentlichen Messen eines Konvektionswärmestroms besteht, einem Heizelement (105), das in wärmeleitendem Kontakt mit dem Körper steht, einen Temperatursensor (104), der thermisch mit dem Körper gekoppelt ist, um die Körpertemperatur $T_{sen}$ zu messen, und einen Feuchtigkeitssensor (107), der mit der Steuereinheit verbunden ist, um eine Feuchtigkeit $p_a$ der Umgebungsluft zu bestimmen, und einen Umgebungstemperatursensor (107), der mit der Steuereinheit verbunden ist, um eine Umgebungstemperatur $T_{air}$ zu messen,
- Betreiben des Heizelements zur Erwärmung des Körpers (120),
- Bestimmen der Temperatur des Körpers $T_{sen}$,
- Bestimmen eines Konvektionswärmestroms $\Phi_{convection}$ für jedes der sechs oder mehr Sensorpaare aus den Messungen des reflektierenden Sensors,
- Bestimmen eines Strahlungswärmestroms $\Phi_{radiation}$ durch Subtraktion der Messwerte des Absorptionssensors und des Reflexionssensors jedes der sechs oder mehr Sensorpaare,
- Bestimmen eines konvektiven Wärmeübergangskoeffizienten $C_{tr}$ auf der Grundlage von $\Phi_{convection}$, $T_{air}$ und $T_{sen}$ oder auf der Grundlage der dem Heizelement bei mehreren Sensorkörpertemperaturen $T_{sen}$ zugeführten Leistung, und
- Bestimmen einer Umgebungsluftgeschwindigkeit $v_{air}$ auf der Grundlage des Wärmeübergangskoeffizienten $C_{tr}$,
- Eingeben der Werte von M Stoffwechselrate, W Arbeit, $I_{cl}$, Isolierung der Kleidung, $f_{cl}$ Oberflächenfaktor der Kleidung und

Bestimmung des Komfortwerts auf der Grundlage:

- der gemessenen Feuchtigkeit $p_a$ der Umgebungsluft,
- der gemessenen Lufttemperatur $T_{air}$,
- der Körpertemperatur $T_{sen}$,
- des Strahlungswärmestroms $\Phi_{radiation}$ und des Konvektionswärmestroms $\Phi_{covection}$ für jedes der sechs oder mehr Sensorpaare
- einer Einzelmessung mit einem Sensor nach einem der Ansprüche 1 und 2, der eine repräsentative Richtcharakteristik und Sichtfeld für die Person an der Position des Sensors aufweist, oder

- einer Reihe von Messungen mit Sensoren nach einem der Ansprüche 1 und 2, die keine repräsentative Richtungsempfindlichkeit und Sichtfeld für die Person an der Position des Sensors aufweisen, wobei der Sensor so umpositioniert wird, dass er in verschiedene Richtungen zu zeigt, und ein Ergebnis aus diesen Messungen berechnet wird.

15. Verfahren nach Anspruch 14, wobei der Komfortwert eine erwartete durchschnittliche Empfindung PMV umfasst, die eine Funktion von Parametern ist, einschließlich einer Stoffwechselrate M, die in W/m$^2$ ausgedrückt werden kann, der effektiven mechanischen Leistung W, die in W/m$^2$ ausgedrückt werden kann, der Bekleidungsisolierung $I_{cl}$, die in K.m$^2$/W ausgedrückt werden kann, die Lufttemperatur $T_{air}$, eine mittlere Strahlungstemperatur $T_{rad}$, die in °C ausgedrückt werden kann, die Umgebungsluftgeschwindigkeit $v_{air}$, die in m/s ausgedrückt werden kann, und die gemessene Luftfeuchtigkeit $p_a$, die durch den Wasserdampfpartialdruck in Pa ausgedrückt kann, wobei diese Parameter miteinander in Beziehung stehen:

$$PMV = [0,303\ e^{(-0.036\ M)} + 0,028] \times [\ [(M-W) - 3,05 \times 10^{-3}\ [5733 - 6,99\ (M-W) - pa] - 0,42\ [(M-W) - 58,15] - 1.7 \times 10^{-5}\ M\ (5867 - pa) - 0,0015\ M\ (34 - T_{air}) - 3,96\ 10^{-8}\ f_{cl}\ ((T_{cl} + 273)^4 - (T_{rad} + 273)^4) - f_{cl}\ Ctr\ (T_{cl} - T_{air})\ ],$$

wobei die Werte M, W, $I_{cl}$ und $f_{cl}$ vom Benutzer eingegeben werden, und wobei $f_{cl}$ ein Oberflächenfaktor der Kleidung und $T_{cl}$ die Oberflächentemperatur der Kleidung ist,

$$T_{cl} = 35,7 - 0,028\ (M-W) - I_{cl}\ [3,96\ 10^{-8}\ f_{cl}\ ((T_{cl} + 273)^4 - (T_{rad} + 273)^4) + f_{cl}\ C_{tr}\ (T_{cl} - T_{air})].$$

**Revendications**

1. Capteur de flux de chaleur (1, 25) comprenant :

   un corps (2, 26) avec six paires de capteurs ou plus, chaque paire consistant en un capteur d'absorption qui absorbe des rayonnements (9, 33, 35) pour mesurer un flux de chaleur par rayonnement et par convection combiné et en un capteur de réflexion qui réfléchit des rayonnements (10, 34, 36) pour mesurer sensiblement un flux de chaleur par convection,
   le corps présentant trois axes de mesure de flux de chaleur, les axes étant sensiblement perpendiculaires, avec deux paires de capteurs par axe orientées sensiblement dans des directions opposées,
   un élément chauffant (12, 47) qui est en contact conducteur de chaleur avec le corps, capable de chauffer le corps uniformément à 2 °C près pour des températures de corps comprises entre 25 et 40 °C à des températures de l'air ambiant comprises entre 20 et 25 °C à des vitesses de l'air < 1 m/s et
   un capteur de température (11, 45) couplé thermiquement au corps pour mesurer la température du corps $T_{sen}$.

2. Capteur de flux de chaleur selon la revendication 1, portant six paires de capteurs ou plus avec un champ de vision combiné (20, 21) entre $2,5\pi$ et $4\pi$ sr,

3. Capteur de flux de chaleur selon la revendication 1 et 2, portant six paires de capteurs ou plus montées avec leurs champs de vision dans des directions opposées, chacune ayant un champ de vision compris entre $2\pi$ et $4\pi$ sr,

4. Système comprenant le capteur de flux de chaleur (1, 25, 100) selon la revendication 1 ou 2 ou 3, une unité de commande (116) et un capteur de température ambiante (13, 46, 107) connecté à l'unité de commande pour mesurer une température ambiante $T_{air}$, l'unité de commande étant adaptée pour :

   - faire fonctionner l'élément chauffant (12, 47, 105) pour chauffer le corps,
   - déterminer ou contrôler la température du corps, $T_{sen}$
   - déterminer à partir des mesures du capteur de réflexion de chacune des six paires de capteurs ou plus, un flux de chaleur par convection $\Phi_{convection}$,
   - déterminer un flux de chaleur par rayonnement $\Phi_{radiation}$ en soustrayant les mesures du capteur d'absorption et du capteur de réflexion de chacune des six paires de capteurs ou plus,
   - déterminer un coefficient de transfert de chaleur par convection $C_{tr}$ sur la base de $\Phi_{convection}$, $T_{air}$ et $T_{sen}$ ou sur la base de la puissance fournie à l'élément chauffant à plusieurs températures du corps de capteur $T_{sen}$,
   - déterminer une vitesse de l'air ambiant $v_{air}$, sur la base du coefficient de transfert de chaleur $C_{tr}$, et
   - déterminer la température par rayonnement $T_{rad}$ à partir de $T_{sen}$ et de $\Phi_{radiation}$.

5. Système selon la revendication 4, où l'unité de commande contrôle la puissance fournie à l'élément chauffant de sorte que le corps du capteur soit maintenu à une température prédéterminée $T_{sen}$.

6. Système selon la revendication 4 ou 5 où le coefficient de transfert de chaleur $C_{tr}$ est déterminé par

$$C_{tr} \quad = \Phi_{convection} / (T_{air} - T_{sen}), \text{ et}$$

la vitesse de l'air $v_{air}$ est déterminée en utilisant une formule empirique de $C_{tr}$, la formule empirique étant valable pour une géométrie du capteur et une direction du flux d'air ambiant, par exemple par :

$$v_{air} \quad = ((C_{tr} - B) / A)^2,$$

où A et B sont des constantes ; où l'unité de commande fournit une indication lorsque la vitesse de l'air $v_{air}$ dépasse une valeur seuil prédéterminée.

7. Système selon la revendication 4, ou 5 ou 6, comprenant en outre un capteur d'humidité (107) connecté à l'unité de commande pour déterminer une humidité $p_a$ de l'air ambiant, l'unité de commande étant adaptée pour déterminer une valeur de confort pour une personne à la position du capteur, sur la base de :

- l'humidité mesurée $p_a$ de l'air ambiant,
- la température mesurée de l'air, $T_{air}$,
- la température du corps, $T_{sen}$,
- pour chacune des six paires de capteurs ou plus, le flux de chaleur par rayonnement $\Phi_{radiation}$, et le flux de chaleur par convection $\Phi_{convection}$.
- une mesure unique avec un capteur selon les revendications 1 ou 2 ou 3 ayant une réponse directionnelle et un champ de vision représentatifs pour la personne à la position du capteur ou
- une mesure unique avec un capteur n'ayant pas une réponse directionnelle et un champ de vision représentatifs pour la personne à la position du capteur, et utilisant des corrections mathématiques telles que des facteurs de surface projetés valables pour la personne à la position du capteur ou
- une série de mesures avec des capteurs selon les revendications 1 ou 2 ou 3, n'ayant pas une réponse directionnelle et un champ de vision représentatifs pour la personne à la position du capteur, en repositionnant le capteur pour pointer dans des directions différentes, et en calculant un résultat à partir de ces mesures

8. Système selon la revendication 7, où la valeur de confort comprend un vote moyen prédit PMV qui est une fonction de paramètres incluant un taux métabolique M, qui peut être exprimé en $W/m^2$, la puissance mécanique effective W, qui peut être exprimée en $W/m^2$, l'isolation de vêtement $I_{cl}$, qui peut être exprimée en $K \cdot m^2/W$, la température de l'air $T_{air}$, une température par rayonnement moyenne $T_{rad}$, qui peut être exprimée en °C, la vitesse de l'air ambiant $v_{air}$, qui peut être exprimée en m/s et l'humidité mesurée $p_a$ qui peut être exprimée par la pression partielle de vapeur d'eau en Pa, ces paramètres étant reliés entre eux par :

$PMV = [0,303 \, e^{(-0,036 \, M)} + 0,028] \times [ [(M-W) - 3,05 \times 10^{-3} [5733 - 6,99 \, (M-W) - p_a] -0,42 \, [(M-W) - 58,15] - 1,7 \times 10^{-5} M \, (5867 - p_a) - 0,0015 \, M \, (34 - T_{air}) - 3,96 \, 10^{-8} \, f_{cl} \, ((T_{cl} + 273)^4 - (T_{rad} + 273)^4) - f_{cl} \, Ctr \, (T_{cl} - T_{air}) ]$,

où les valeurs de M, W, $I_{cl}$, $f_{cl}$ sont entrées par l'utilisateur, et $f_{cl}$ est un facteur de surface de vêtement, et $T_{cl}$ la température de la surface de vêtement :

$T_{cl} = 35,7 - 0,028 \, (M-W) - I_{cl} \, [3,96 \, 10^{-8} \, f_{cl} \, ((T_{cl} + 273)^4 - (T_{rad} + 273)^4) + f_{cl} \, Ctr \, (T_{cl} - Tair)]$

9. Système selon la revendication 4, où l'unité de commande contrôle la puissance fournie à l'élément chauffant de sorte que le corps du capteur soit maintenu à $T_{cl}$ et les valeurs PMV et PPD sont des mesures à ou autour de $T_{cl}$

10. Système selon l'une quelconque des revendications 4 à 9, l'unité de commande étant adaptée pour comparer la puissance de chauffage totale fournie au corps pour maintenir le corps à la température prédéterminée, avec le flux de chaleur mesuré par le capteur de réflexion et le flux de chaleur mesuré par le capteur d'absorption de chacune des six paires de capteurs ou plus.

**11.** Système selon l'une quelconque des revendications 4 à 10, l'unité de commande chauffant le corps à une ou plusieurs températures stables par exemple $T_{cl}$, déterminant ainsi la température de fonctionnement $T_{operative}$ :

$$T_{operative} = T_{cl} + ( \Phi_{radiation, \, cl} + \Phi_{convection, \, cl} ) / (C_{tr} + C_{rad})$$

avec l'indice "cl" indiquant la température de surface de vêtement et $C_{rad}$ le coefficient de transfert de chaleur par rayonnement.

**12.** Système selon l'une quelconque des revendications 4 à 11, l'unité de commande chauffant le corps à un certain nombre de températures différentes, et déterminant à partir des flux de chaleur par rayonnement $\Phi_{radiation}$ et des flux de chaleur par convection $\Phi_{convection}$ à ces températures une température de l'air, des coefficients de transfert de chaleur par rayonnement et par convection, et une température de fonctionnement.

**13.** Système selon les revendications 8 ou 9 en combinaison avec la revendication 2, ayant 2 ou un autre nombre pair de paires de capteurs, où l'unité de commande détermine une valeur de confort, un flux de chaleur par rayonnement ou un flux de chaleur par convection pour chaque surface, et fournit une indication si une différence de valeur de confort, de flux de chaleur par rayonnement ou de flux de chaleur par convection est supérieure à une valeur seuil prédéterminée.

**14.** Procédé de détermination d'une valeur de confort, en particulier d'un vote moyen prédit PMV, le procédé comprenant les étapes de :

- fournir un capteur de flux de chaleur (100) ayant un corps (99) avec six paires de capteurs ou plus, chaque paire de capteurs consistant en un capteur d'absorption (101) pour mesurer un flux de chaleur par rayonnement et par convection combiné et un capteur de réflexion (102) pour mesurer sensiblement un flux de chaleur par convection, un élément chauffant (105) qui est en contact conducteur de chaleur avec le corps, un capteur de température (104) couplé thermiquement au corps pour mesurer la température du corps $T_{sen}$, et un capteur d'humidité (107) connecté à l'unité de commande pour déterminer une humidité $p_a$ de l'air ambiant, et un capteur de température ambiante (107) connecté à l'unité de commande pour mesurer une température ambiante $T_{air}$,
- faire fonctionner l'élément chauffant pour chauffer le corps (120),
- déterminer la température du corps, $T_{sen}$,
- déterminer pour chacune des six paires de capteurs ou plus, à partir des mesures du capteur de réflexion, un flux de chaleur par convection $\Phi_{convection}$,
- déterminer pour chacune des six paires de capteurs ou plus un flux de chaleur par rayonnement $\Phi_{radiation}$ en soustrayant les mesures du capteur d'absorption et du capteur de réflexion,
- déterminer un coefficient de transfert de chaleur par convection $C_{tr}$ sur la base de $\Phi_{convection}$, $T_{air}$ et $T_{sen}$ ou sur la base de la puissance fournie à l'élément chauffant à plusieurs températures du corps de capteur $T_{sen}$, et
- déterminer une vitesse de l'air ambiant $v_{air}$, sur la base du coefficient de transfert de chaleur $C_{tr}$,
- entrer des valeurs de taux métabolique M, travail W, isolation de vêtement $I_{cl}$, facteur de surface de vêtement $f_{cl}$ et
- déterminer la valeur de confort sur la base de :

- l'humidité mesurée $p_a$ de l'air ambiant,
- la température mesurée de l'air, $T_{air}$,
- la température du corps, $T_{sen}$,
- pour chacune des six paires de capteurs ou plus le flux de chaleur par rayonnement $\Phi_{radiation}$, et le flux de chaleur par convection $\Phi_{convection}$.
- une mesure unique avec un capteur selon les revendications 1 et 2 ayant une réponse directionnelle et un champ de vision représentatifs pour la personne à la position du capteur ou
- une série de mesures avec des capteurs selon les revendications 1 et 2 n'ayant pas une réponse directionnelle et un champ de vision représentatifs pour la personne à la position du capteur, en repositionnant le capteur pour pointer dans des directions différentes, et en calculant un résultat à partir de ces mesures

**15.** Procédé selon la revendication 14, où la valeur de confort comprend un vote moyen prédit PMV qui est une fonction de paramètres incluant un taux métabolique M, qui peut être exprimé en $W/m^2$, la puissance mécanique effective W, qui peut être exprimée en $W/m^2$, l'isolation de vêtement $I_{cl}$, qui peut être exprimée en $K \cdot m^2/W$, la température de l'air $T_{air}$,

une température par rayonnement moyenne $T_{rad}$, qui peut être exprimée en °C, la vitesse de l'air ambiant $v_{air}$, qui peut être exprimée en m/s et l'humidité mesurée $p_a$ qui peut être exprimée par la pression partielle de vapeur d'eau en Pa, ces paramètres étant reliés entre eux par :

$$PMV = [0{,}303\, e^{(-0{,}036\, M)} + 0{,}028] \times [\, [(M\text{-}W) - 3{,}05 \times 10^{-3} [5733 - 6{,}99\,(M\text{-}W) - p_a] - 0{,}42\,[(M\text{-}W) - 58{,}15] - 1{,}7 \times 10^{-5} M\,(5867 - p_a) - 0{,}0015\,M\,(34 - T_{air}) - 3{,}96\,10^{-8}\, f_{cl}\,((T_{cl} + 273)^4 - (T_{rad} + 273)^4) - f_{cl}\,Ctr\,(T_{cl} - T_{air})\,],$$

où les valeurs de M, W, $I_{cl}$, $f_{cl}$ sont entrées par l'utilisateur, et $f_{cl}$ est un facteur de surface de vêtement, et $T_{cl}$ la température de surface de vêtement

$$T_{cl} = 35{,}7 - 0{,}028\,(M\text{-}W) - I_{cl}\,[3{,}96\,10^{-8}\, f_{cl}\,((T_{cl} + 273)^4 - (T_{rad} + 273)^4) + f_{cl}\,Ctr\,(T_{cl} - Tair)]$$

# Fig. 1

13    10, 15, 17

9, 14, 16

3

12

11

6

7

8

5

4

2

1

# Fig. 2

9

10

14

15

18

19

16

17

3

# Fig. 3

20, 21

9, 14

10, 15

3

# Fig. 4

51

1

3

9, 14

10, 15

53

52

# Fig. 5

# Fig. 6

# Fig. 7

120 — Power heater

121 — Store $T_{sen}$

122 —
- Measure and store $S_{ab}$, $S_{ref}$, $T_{air}$ and $p_a$
- Determine $\Phi_{convection} = S_{ref}$
  $\Phi_{radiation} = S_{ab} - S_{ref}$
- Determine $C_{tr}$ from formula [1]
- Determine $T_{rad}$ from formula [2]
- Deteremine $v_{air}$ form formula [3]

124

123 — $v_{air} > 1m/s$ — Give indication on terminal 116

125 —
Read input values for
$M$, $I_{cl}$, $f_{cl}$, $W$ at terminal 116

No

127

126 — Calculate $T_{cl}$ from formula [5] — $T_{sen} = T_{cl}$

Yes

128 —
- Calculate PMV from formula [4]
- Deteremine $T_{operative}$ from formula

129 — Store and/or display PMV and $T_{operative}$

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200189352 A